# FASCICULE DE BREVET EUROPEEN

(11) **EP 1 730 159 B1**
(45) Date de publication et mention de la délivrance du brevet: **03.10.2007**
(21) Numéro de dépôt: 05736945.6
(22) Date de dépôt: 11.03.2005
(51) Int. Cl.: C07H 17/07, A61K 31/704

(54) **DERIVES DE GENKWANINE ET SAKURANETINE, UTILISATION COSMETIQUE ET THERAPEUTIQUE, ET PROCEDE DE PREPARATION**
DERIVATE VON GENKWANIN UND SAKURANETIN, DEREN KOSMETISCHE UND THERAPEUTISCHE VERWENDUNG, UND VERFAHREN ZU DEREN HERSTELLUNG
DERIVATIVES OF GENKWANIN AND SAKURANETIN, COSMETIC AND THERAPEUTIC USE THEREOF AND PREPARATION METHOD OF SAME

(30) Priorité: 11.03.2004 FR 0402554
(43) Date de publication de la demande: 13.12.2006
(73) Titulaire: Prost, Michel, 21560 Couternon (FR); Ragot, Jacqueline, 31400 Toulouse (FR); Tubery, Pierre, 31600 Lamasquère (FR)
(72) Inventeur: Prost, Michel, 21560 Couternon (FR); Ragot, Jacqueline, 31400 Toulouse (FR); Tubery, Pierre, 31600 Lamasquère (FR)
(74) Mandataire: Bertrand, Didier
(86) Numéro de dépôt international: PCT/FR2005/000596
(87) Numéro de publication internationale: WO 2005/087786

(56) Documents cités:
- EP-A- 0 633 022
- WO-A-03/031430
- FR-A- 2 597 751
- DEIANA M ET AL: "CHEMICAL COMPOSITION AND ANTIOXIDANT ACTIVITY OF EXTRACTS FROM DAPHNE GNIDIUM L" JOURNAL OF THE AMERICAN OIL CHEMISTS' SOCIETY, AMERICAN OIL CHEMISTS' SOCIETY. CHAMPAIGN, US, vol. 80, no. 1, 2003, pages 65-70, XP001183469 ISSN: 0003-021X

## Description

### Domaine de l'invention

La présente invention a trait à des dérivés osylés de genkwanine et sakuranétine. Plus précisément, elle concerne (i) l'utilisation en cosmétique ou dermatologie, d'une part, et en thérapeutique, d'autre part, des dérivés osylés de genkwanine et sakuranétine de formule I ci-après, (ii) des nouveaux dérivés de formule I en tant que produits industriels, et (iii) leur procédé de fabrication.

Les composés selon l'invention répondent à la formule I : 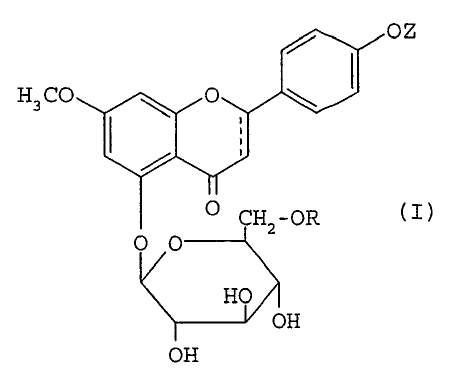 dans laquelle,
le symbole ---- représente une simple ou une double liaison,
R représente H ou un reste osyle, notamment de structure S¹ ou S² : 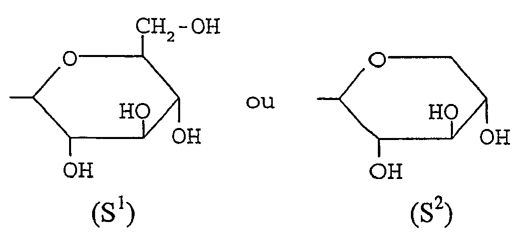
Z représente H, un groupe alkyle en C₁-C₄, acyle en C₁-C₅, ose ou sulfate.

### Art antérieur

On sait que quelques produits de formule I ont déjà été décrits et étudiés dans le passe (cf . EP 0 633 022 ou encore Deiana M. et al., JAOCS, Vol 80 (1), 2003). En particulier la 5-O-β-D-primevérosyl-genkwanine (qui est un composé de formule I où le symbole ---- représente une double liaison, R est un reste osyle de structure S², et Z est H) est obtenue par extraction de ***Gnidia kraussiana*** (une plante de la savane africaine de la famille des thyméléacées) et possède des propriétés immunitaires (notamment immunostimulantes), anticancéreuses et antileucémiques. Plus précisément, lors de désordres immunitaires graves, les lymphoblastes physiologiques sont en hyperplasie, et l'intérêt de la 5-O-β-D-primevérosylgenkwanine réside dans le fait qu'elle détruit les lymphoblastes formés. Voir à cet effet FR 2510580 A, FR 2597751 A et l'article de Jer-Huei LIN et al., Yaowu Shipin Fenxi, 2001; 9(1), 6-11*.*

Le pinostrobine-5-glucoside (qui est un composé de formule I où le symbole ---- représente une double liaison, R est H, et Z est H) a été isolé de l'écorce de ***Prunus cerasus*** et est considéré comme étant caractéristique de l'espèce ***Prunus cerasus**.* Voir à cet effet l'article de Martin GEIBEL et al., Phythochemistry, 1991;30(5),1519-1521*.*

La sakuranine, autre nomenclature : sakuranétine-5-glucoside, (qui est un composé de formule I où le symbole ---- représente une simple liaison, R est H, et Z est H), a été isolée de ***Prunus yedoensis*** sans que ses éventuelles propriétés cosmétiques ou pharmacologiques (notamment les propriétés antiradicalaires) aient été étudiées. Voir à cet effet l'ouvrage Merck Index, 12th Edition, 1996, Monograph No. 8470, pages 1431-1432*.*

L'art antérieur précité ne décrit ni ne suggère que les composés de formule I selon l'invention présentent des propriétés bénéfiques :
- en cosmétique ou dermatopharmacie, en tant que substances améliorant la texture de la peau, et
- en thérapeutique humaine ou vétérinaire (notamment les animaux à sang chaud), en tant que substances antiradicalaires.

Notons toutefois que WO 03/031430 décrit également des flavonoïdes capables d'enterager avec des membranes biologiques.

### Objet de l'invention

Selon un premier aspect de l'invention, on préconise une nouvelle utilisation de dérivés osylés de genkwanine et sakuranétine, en tant que substances (a) cosmétiques ou dermatologiques, ou (b) antiradicalaires, pour (a) améliorer la texture de la peau ou, respectivement, (b) traiter ou prévenir les désordres provoqués par les radicaux libres.

Dans cette optique, on fournit une nouvelle utilisation (a) en cosmétique ou dermatologie, d'une part, ou (b) en thérapeutique humaine ou vétérinaire, d'autre part, ladite utilisation étant caractérisée en ce que l'on fait appel à une substance choisie parmi l'ensemble constitué par
(i) les dérivés osylés de genkwanine ou sakuranétine de formule I : 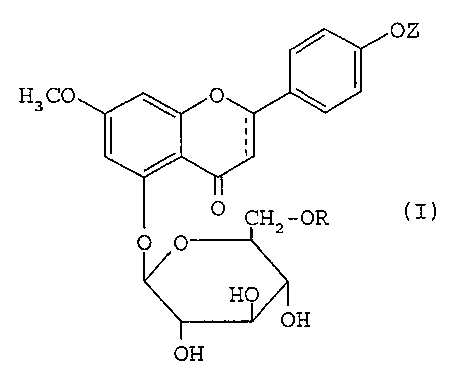 dans laquelle,
   le symbole ---- représente une simple ou une double liaison,
   R représente H ou un reste osyle, notamment de structure S¹ ou S² : 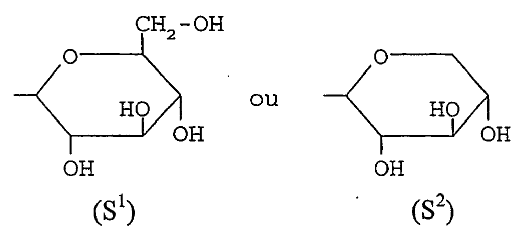
   Z représente H, un groupe alkyle en C₁-C₄, acyle en C₁-C₅, ose ou sulfate, et
(ii) leurs mélanges,
en tant qu'ingrédient actif (a) cosmétique ou dermatologique, ou, respectivement, (b) antiradicalaire, pour l'obtention (a) d'une préparation cosmétique ou dermatologique pour améliorer la texture de la peau ou, respectivement (b) d'un médicament destiné à un usage en thérapeutique vis-à-vis des désordres provoqués par les radicaux libres.

Selon un second aspect de l'invention, on préconise, en tant que produits industriels nouveaux, des composés de formule I où R est notamment un reste ose de structure S¹ et leurs mélanges.

Selon un troisième aspect de l'invention, on préconise un procédé pour la préparation des composés de formule I et en particulier pour celle desdits nouveaux composés.

### Brève description des dessins

Les figures annexées concernent une partie des résultats des essais qui ont été entrepris avec des produits de formule I :
- la figure 1 montre que les produits de formule I testés ont des propriétés antiradicalaires, et
- les figures 2 et 3 montrent que les produits de formule I testés présentent un intérêt en tant qu'agents agents immunosuppresseurs.

### Description détaillée de l'invention

La présente invention englobe des dérivés osylés de genkwanine quand le symbole ---- représente une double liaison, d'une part, et des dérivés osylés de sakuranétine quand ledit symbole ---- représente une simple liaison, d'autre part.

Dans la définition de Z, les groupe alkyle en C₁-C₄ comprennent des groupes à chaîne hydrocarbonée linéaire ou ramifiée, à savoir les groupes méthyle, éthyle, n-propyle, i-propyle, n-butyle, sec.-butyle et tert.-butyle ; les groupes acyle en C₁-C₅ comprennent les groupes aliphatiques à chaîne hydrocarbonée linéaire ou ramifiée ayant de 1 à 5 atomes de carbone, à savoir les groupes CH₃CO, CH₃CH₂CO, CH₃CH₂CH₂CO, (CH₃)₂CHCO, CH₃CH₂CH₂CH₂CO, (CH₃)₂CHCH₂CO, CH₃CH₂CH(CH₃)CO et (CH₃)₃CCO ; le groupe sulfate comprend le reste SO₃⁻ que l'on rencontre principalement sous la forme acide SO₃H et, le cas échéant, sous une forme salifiée telle que SO₃NH₄ ou SO₃Na. Enfin le groupe Z peut représenter un reste osyle, notamment un reste glucosyle, xylosyle, thioxylosyle, fructosyle, mannosyle, etc.

Le groupe osyle, qui intervient dans la définition de R, peut être un reste ose quelconque, notamment un des restes énumérés plus haut pour le groupe pour Z. Avantageusement, les groupes R selon l'invention seront de structure S¹ ou S², la structure S¹ étant la préférée.

Parmi les composés de formule I conformes à l'invention, l'on peut avantageusement citer :
- la 5-[O-6-(D-glucopyranosyl)-β-D-glucopyranosyl]oxy-2-(4-éthoxyphényl)-7-méthoxy-4H-1-benzopyran-4-one [autre nomenclature : 4'-éthoxygenkwanine-5-(D-glucosido)-p-D-glucoside] de formule Ia : 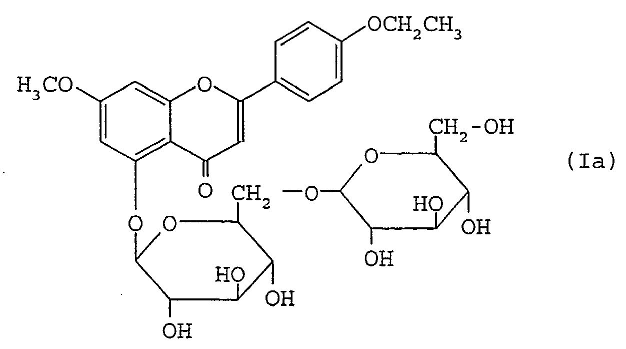 qui est le produit le plus intéressant de l'invention ;
- la 5-O-β-D-primevérosyl-genkwanine précitée de formule IIa : 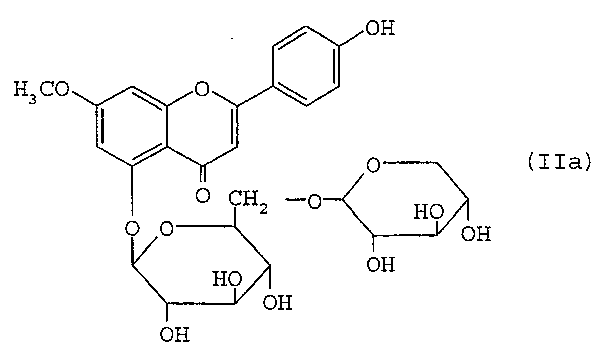
- le pinostrobine-5-glucoside précité de formule IIIa : 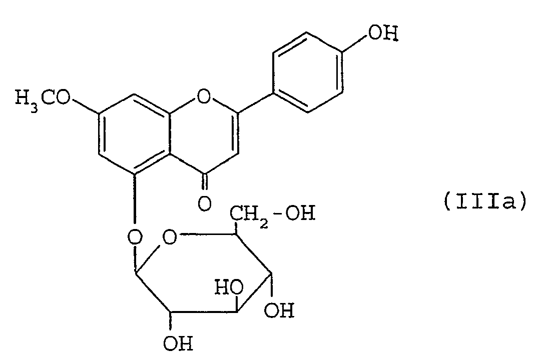
- la 2,3-dihydro-5-[O-6-(D-glucopyranosyl)-β -D-glucopyranosyl]oxy-2-(4-éthoxyphényl)-7-méthoxy-4H-1-benzopyran-4-one [autre nomenclature : 4'-éthoxysakuranétine-5-(D-glucosido)-β-D-glucoside de formule Ib : 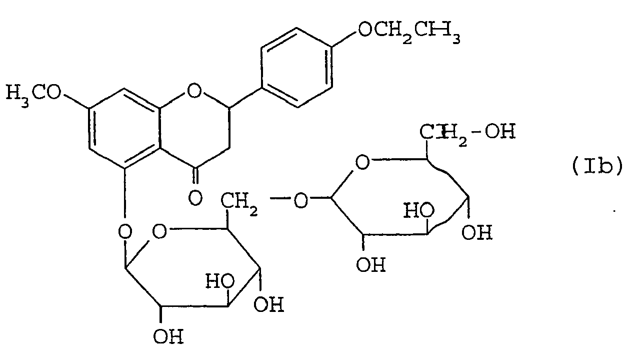 qui est l'homologue du produit de formule Ia eu égard au remplacement de la genkwanine par la sakuranétine,
- la 5-O-β-D-primevérosyl-sakuranétine de formule IIb : 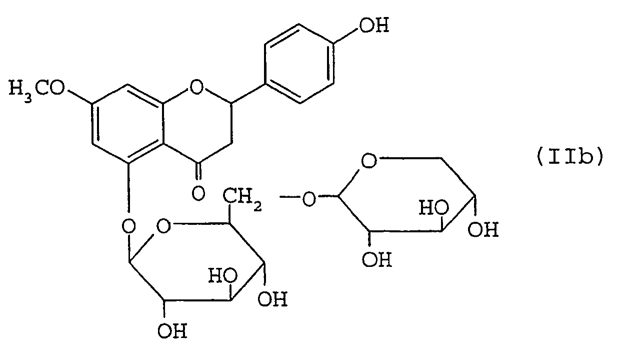 et leurs dérivés où Z est un groupe sulfate (de préférence SO₃H, ou le cas échéant SO₃Na voire SO₃NH₄).

Parmi les composés nouveaux selon l'invention, l'on peut citer plus particulièrement les produits de formule IV : 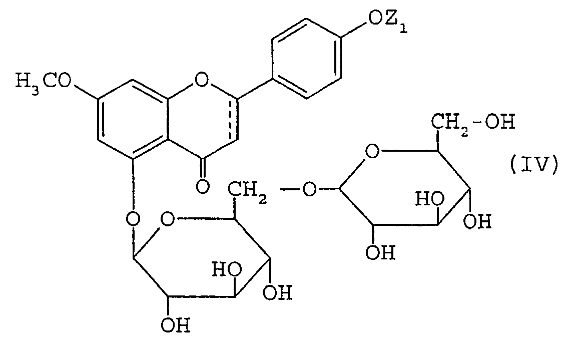 dans laquelle symbole ---- représente une simple ou une double liaison et Z₁ a la même définition que Z ci-dessus et représente avantageusement un groupe alkyle en C₁-C₄ (de préférence le groupe éthyle) ou sulfate (de préférence le groupe SO₃H).

Les composés de formule I peuvent être préparés selon une méthode connue en soi par application de mécanismes réactionnels et/ou processus d'extraction classiques. A titre d'exemple : (i) on extrait la genkwanine, la sakuranétine ou un de leurs osides à partir d'une plante appropriée appartenant à l'ensemble des ***Prunus, Gnidia*** et ***Daphne* ;** (ii) on osyle en position 5 l'aglycone avec un ose approprié (si nécessaire après blocage de la fonction OH en position 4' si celle-ci n'est pas protégée), et/ou (iii) on éthérifie (notamment au moyen d'un iodure d'alkyle pour ne pas affecter les groupes OH de la portion sucre), estérifie ou sulfate le groupe 4'-OH de l'oside extrait ou préparé comme indiqué ci-dessus (si nécessaire après déprotection du groupe 4'-OH).

Le procédé, que l'on recommande selon l'invention pour préparer le composé de formule Ia, est caractérisé en ce qu'il comprend les étapes consistant à :
(1°) extraire les racines broyées de ***Daphne gnidium*** avec CH₂Cl₂,
(2°) filtrer pour écarter la solution de chlorure de méthylène, ainsi obtenue, et recueillir le résidu solide que l'on sèche ;
(3°) extraire ledit résidu solide sec, ainsi obtenu, avec CH₃OH ;
(4°) filtrer pour recueillir la solution méthanolique, ainsi obtenue, et écarter le résidu solide résultant ;
(5°) évaporer à sec la solution méthanolique, ainsi recueillie, sous vide, à une température inférieure ou égale à 60 °C, pour obtenir un résidu solide ;
(6°) laver le résidu solide, ainsi obtenu à l'étape (5°), avec de l'eau à une température inférieure ou égale à 60 °C sous agitation, et laisser refroidir ;
(7°) éliminer l'eau de lavage puis reprendre le résidu solide avec CH₃OH ;
(8°) reproduire 3 à 7 fois le cycle des opérations des étapes (5°) à (7°) jusqu'à ce que la dernière eau de lavage soit jaune pâle et limpide ;
(9°) reprendre le résidu sec résultant avec un mélange méthanol-eau 25/2 p/p selon une quantité appropriée pour obtenir un liquide ayant une densité de 0,885 g/mL ;
(10°) laisser reposer ledit liquide à 2-4 °C, de préférence à 3 °C, pendant au moins 2 jours, de préférence pendant 3 jours, et recueillir le précipité formé ;
(11°) laver ledit précipité avec successivement du méthanol puis des mélanges méthanol-éther ayant des teneurs croissantes en éther, jusqu'à ce que le surnageant soit incolore ;
(12°) filtrer le précipité, ainsi obtenu, et le laver plusieurs fois à l'éther, jusqu'à ce que l'éther de lavage soit incolore ;
(13°) filtrer et sécher le produit solide résultant qui est constitué d'un mélange des produits de formules Ia, IIa et IIIa ; et,
(14°) si nécessaire, séparer ledit mélange pour recueillir le produit de formule Ia.

De façon pratique, l'extraction de l'étape (1°) est menée à chaud (i. e. à une température de 30-35 °C sous pression atmosphérique (≈ 10⁵ Pa) ou, le cas échéant, à une température plus élevée sous pression réduite) pendant 3-6 jours (de préférence pendant 5 jours) dans un appareil de type Kumagawa ; l'extraction de l'étape (3°) est effectuée à chaud (notamment à une température de 45-55 +C sous pression normale (≈ 10⁵ Pa) ou, le cas échéant, à une température plus élevée sous pression réduite) dans le même appareil pendant 3-6 jours (de préférence pendant 5 jours).

Eu égard aux modalités préférentielles sus-visées, on obtient à l'issue de l'étape (13°) un mélange Ia/IIa/IIIa selon un rapport pondéral d'environ 10/85/5 p/p.

En fonction des purifications entreprises par chromatographie, on obtient à l'issue de l'étape (14°) :
- un mélange Ia/IIa enrichi en Ia, notamment le mélange Ia/IIa 80/20 p/p, ou
- le composé de formule Ia essentiellement pur (i. e. ayant une pureté supérieure ou égale à 98 %) ou plus purifié (i. e. ayant une pureté supérieure ou égale à 99,5 %).

Les composés de formule I, et en particulier les composés nouveaux de formule IV, sont utiles en cosmétique ou dermatopharmacie en tant qu'agents améliorant la texture de la peau.

Administrés par voie topique, sous forme d'une solution, d'une lotion, d'un gel ou d'une émulsion le cas échéant multiple (par exemple une émulsion H/L/H ou L/H/L), les composés de formule I ou IV ont :
- une action favorable sur les effets du vieillissement de la peau, notamment pour réduire les rides et assurer la fermeté et la souplesse souhaitées pour la peau ;
- un effet anti-age permettant d'éviter l'injection de collagène ; et,
- un pouvoir de contrôle de l'hydratation de la peau.

En particulier, comme les composés de formule I ou, respectivement, IV s'hydratent aisément en I.xH₂O ou, respectivement, IV.xH₂O (où x est un nombre entier ou fractionnaire compris notamment entre 0,3 et 5), ils servent, selon l'invention, dans l'épaisseur de la peau en tant que régulateurs d'hydratation, soit en captant l'eau en excès, soit surtout en apportant de l'eau quand la teneur en eau dans la peau est insuffisante.

Outre l'aspect cosmétique ou dermatologique précité, les composés de formule 1 ou IV sont utiles en thérapeutique humaine et vétérinaire en raison de leurs propriétés antiradicalaires pour traiter et surtout prévenir les désordres induits par les radicaux libres.

Lesdits désordres comprennent en particulier les pathologies induites par une hyperproduction ou une production non contrôlée de radicaux libres dans l'organisme, telles que les maladies myélodégénératives, le syndrome maniaco-dépressif, et la démence sénile. Les composés de formules I ou IV sont surtout intéressants en thérapeutique humaine avant que ces pathologies ne deviennent irréversibles

Par ailleurs, tous les composés de formule IV, qui ont été testés en vue de leurs propriétés immunomodulatrices, antiathéromateuses et anticancéreuses, se sont révélés efficaces. La substance préférée selon l'invention, qui est constituée par le produit de formule Ia ou les mélanges Ia/IIa/IIIa (i. e. extrait de ***Daphne gnidium**)* et Ia/IIa précités, est particulièrement active vis-à-vis de certains cancers et leucémies aiguës (effet antiblastique, i. e. destruction des leucoblastes), et de la leucémie myéloïde chronique.

Selon l'invention, l'on préconise une composition cosmétique (a), dermatopharmaceutique (b) ou thérapeutique (c), qui est caractérisée en ce que :
(a) la composition cosmétique renferme, en association avec un excipient physiologiquement acceptable par voie topique, au moins un composé de formule I ;
(b) la composition dermatopharmaceutique renferme, en association avec un excipient physiologiquement acceptable notamment par voie topique, au moins un composé de formule I ; ou,
(c) la composition thérapeutique renferme, en association avec un excipient physiologiquement acceptable notamment par voie orale ou injectable, au moins un composé de formule IV en tant qu'ingrédient actif immunomodulateur, notamment vis-à-vis de poussées récentes de sclérose en plaque, ou anticancéreux, notamment vis-à-vis de la leucémie myéloïde chronique.

D'autres avantages et caractéristiques de l'invention seront mieux compris à la lecture qui va suivre d'exemples de préparation et de résultats d'essais cosmétologiques et pharmacologiques. Bien entendu, l'ensemble de ces éléments n'est nullement limitatif mais est fourni à titre d'illustration.

### Exemples

Quelques composés typiques de formule I ont été consignés dans le tableau I ci-après avec des produits de comparaison (CP.1 et CP.2).

**Tableau I**

| ***Composés typiques selon l'invention*** | |
|---|---|
| Exemple | Structure |
| Ex. 1 | mélange IalIla/IIla 10/85/5 p/p |
| Ex. 2 | produit de formule IIa |
| Ex. 3 | produit de formule IIIa |
| Ex. 4 | mélange la/IIa 80/20 p/p |
| Ex. 5 | produit de formule Ib |
| Ex. 6 | produit de formule IIb |
| Ex. 7 | 4'-sulfate du produit de formule Ib |
| Ex. 8 | Produit de formule Ia |
| Ex. 9 | 4'-sulfate du produit de formule Ia |
| Ex. 10 | mélange Ib/IIa/IIIa 10/85/5 p/p |
| CP.1 | genkwanine |
| CP.2 | sakuranétine |

### Préparation A

### - Obtention du mélange Ia/IIa/Illa 10/85/5 p/p (Ex. 1) -

11 kg de racines de ***Daphne gnidium*** (plante du bassin méditerranéen de la famille des thyméléacées) sont broyés puis traités en continu avec du chlorure de méthylène, à 30-35 °C, pendant 5 jours dans un appareil de type Kumagawa. La solution liquide ainsi obtenue est écartée et on recueille le résidu solide que l'on sèche. On extrait ledit résidu, ainsi séché, avec du méthanol à chaud ( 45-55 °C) pendant 5 jours dans ledit appareil de type Kumagawa. L'extrait méthanolique, obtenu après avoir écarté le résidu solide, est traité de la façon suivante : évaporation à sec sous pression réduite à une température inférieure à 60 °C dans un ballon ; lavage du résidu solide, ainsi obtenu, à l'eau chaude en agitant de façon à décoller ledit résidu du fond du ballon ; refroidissement à la température ambiante et élimination de l'eau de lavage ; et reprise du résidu dans du méthanol. Cette succession de traitements est reproduite de 5 à 7 fois, selon l'origine des racines, jusqu'à ce que la dernière eau de lavage soit limpide et de couleur jaune pâle. Le résidu résultant est repris à chaud (45-55 °C) avec du méthanol contenant 8 % en poids d'eau, en quantité suffisante pour obtenir un liquide ayant une densité de 0,885 g/mL. On laisse reposer pendant 3 jours à 3 °C puis récupère le précipité formé par centrifugation. On lave ce précipité par fractions successives de méthanol puis de mélanges méthanol/diméthyl éther (ou méthanol/diéthyl éther) de plus en plus riches en éther. Quand le surnageant est enfin presque incolore, on filtre le précipité et le lave plusieurs fois à l'éther jusqu'à ce que l'éther de lavage soit incolore. On obtient un solide beige très clair que l'on sèche sous pression réduite puis qui est broyé.

Ce solide est un mélange Ia/IIa/IIIa dans un rapport pondéral de 10/85/5. Le rendement est de l'ordre de 2 à 3 % selon l'origine de la plante et la saison de la récolte des racines.

### Analyse

Les composés de formules Ia, IIa et IIIa étant de structures proches (partie favonoide et partie saccharidique) ont de fortes similarités spectroscopiques, en particulier en ultra-violet et en infra-rouge.

### Spectre UV (dans mélange acétonirile-eau 80/20 v/v)

On observe deux bandes d'absorption à 331,7 et 261,7 nanomètres (la bande à 261,7 nm ayant une intensité qui est d'environ la moitié de celle de la bande à 331,7 nm)..

### Spectres IR (en pastille de KBr)

On observe les bandes suivantes:
- bande intense à 3374 cm⁻¹ (O-H de la partie sucre);
- bande intense à 1635 cm⁻¹ (bande de vibration du carbonyle de la flavone);
- bande d'intensité moyenne à 1609 cm⁻¹ (bande de vibration de la double liaison éthylénique de la flavone) ; et
- bandes d'intensité moyenne à 1450 et 1360 cm⁻¹ (bandes de vibration des parties aromatiques).

### Préparation B

### - Obtention du mélange Ia/IIa 80/20 p/p (Ex. 4) -

En soumettant le produit de l'exemple 1 à une chromatographie séparative (HPLC), on obtient le mélange Ia/IIa 80/20 p/p.

### Préparation C

### - Obtention du produit de formule Ia (Ex. 8) -

En soumettant le produit de l'exemple 1 ou de l'exemple 4 à une chromatographie séparative plus poussée, on obtient le composé de formule Ia avec une pureté supérieure ou égale à 98 % , voire même avec une pureté supérieure ou égale à 99,5 %.

### Analyse

On a déterminé les spectre de RMN (à 250 Mhz en solution dans du méthanol deutéré) et spectre de masse (par technique FAB). Les résultats obtenus sont les suivants, où la 1^{ère} unité sucre est celle qui est liée au squelette flavone et la 2^{ème} unité sucre est celle de structure S¹ ou S².

### Spectre RMN

- triplet centré à 1,31 ppm (groupe méthyle CH₃ de la chaîne phénolique alkylée),
- quadruplet centré à 3,20 ppm (groupe méthylène CH₂ de ladite chaîne alkylée) ;
- massif de 3,27 à 4,39 ppm (protons des deux unités sucre) [attributions détaillées sur la base d'expériences COSY, HMQC et HMBC à 600 Mhz, dont les deux protons anomères des deux unités sucre, respectivement à 4,75 ppm (doublet) pour la 1^{ère} unité liée en position 5 à la flavone, et à 4, 27 ppm (doublet) pour la 2^{ème} unité ; pont -CH₂-O- entre les deux unités sucre à 3,60 (d) et 3,93 (d) ppm ; et -CH₂- en 5 sur 2^{ème} unité sucre à 3,32 (d) et 3,60 (d) ppm ; la stéréochimie dans les deux unités sucre ayant été établie sur la base des couplages proton-proton vicinaux en partant des protons anomères] ;
- 3,87 ppm (CH₃ du groupement CH₃-O-) ;
- 6,60 ppm (proton éthylénique de la partie flavone);
- massif 6,91-6,94 ppm (4 protons aromatiques) ; et
- massif 7,82-7,86 ppm (2 protons aromatiques).

### Spectre de masse

Masse moléculaire : 636,598 (C₃₀H₃₆O₁₅)

Pic de masse : 636 ; adduits de Na et K conformes.

La méthode de spectrométrie de masse a été également utilisée pour confirmer les structures de formules Ia, IIa et IIIa après acétylation de tous les groupements O-H (par le mélange anhydride acétique/pyridine) ; il a été procédé à l'analyse par spectrométrie de masse des produits d'acétylation après purification chromatographique sur silice (éluant : eau/acétonitrile 50/50 v/v).

### Préparation C bis

### - Obtention des produits de formule IIa (Ex. 2) et de formule IIIa (Ex. 3) -

En soumettant le produit de l'exemple 1 à des chromatographies séparatives plus poussées, on a isolé les produits de formule IIa (Ex. 2) et de formule IIIa (Ex. 3) avec une pureté supérieure ou égale à 98 %.

### Analyse (entreprise comme indiqué à la préparation C ci-dessus)

### Spectre RMN de Ex. 2

Le spectre de RMN du produit de formule IIa (Ex. 2) est identique à celui du produit de formule Ia (Ex. 8) aux différences près suivantes :
- absence des signaux CH₃ à 1,31 ppm et CH₂ à 3,20 ppm de la chaîne éthyle ;
- disparition des signaux à 3,32 et 3,60 ppm du CH₂ en position 5 sur le second sucre.

### Spectre de masse de Ex. 2

Masse moléculaire : 578,519 (C₂₇H₃₀O₁₄)

Pic de masse : 578 ; adduits de Na et K conformes.

### Spectre RMN de Ex. 3

Le spectre de RMN du produit de formule IIIa (Ex. 3) est identique à celui du produit de formule la (Ex. 8) à la différence près suivantes :
- simplification du massif correspondant aux protons de la partie sucre avec un seul proton anomère à 4,76 ppm (d).

### Spectre de masse de Ex. 3

Masse moléculaire : 446,404 (C₂₂H₂₂O₁₀)

Pic de masse : 446 ; adduits de Na et K conformes.

### Préparation D

### - Obtention du produit de formule Ib (Ex.5) -

En reproduisant le procédé des Préparations A et C ci-dessus, à partir de l'écorce ou des racines de ***Prunus yedoensis,*** on obtient le composé de formule Ib.

### Préparation E

### - Obtention du 4'-sulfate du produit de formule Ib (Ex.7) -

Le produit attendu est obtenu par sulfatation du groupe 4'-OH selon une méthode connue en soi.

### Essais F

La propriété d'améliorer la texture de la peau a été appréciée par le biais de la régénération du tissu cutané après brûlure.

On rase une portion du dos de rats mâles adultes et applique sur cette portion une platine métallique de 0,5 cm² chauffée à une température de 130 °C pour créer une zone de brûlure calibrée. On applique un gel contenant 0 (lot témoin) ou 1,5 % en poids de produit de formule I (lots traités) une fois par jour pendant 21 jours sur la brûlure des rats (8 animaux par produit à tester, 10 animaux pour le lot témoin). On constate que, chez les lots traités (Ex 1 à Ex 10), la régénération du tissu cutané est obtenue en 1 mois ; en revanche chez le lot témoin, ladite régénération intervient en 6 à 8 semaines

### Essais G

L'étude des propriétés antiradicalaires des produits selon l'invention (Ex. 1 à Ex. 10) a été entreprise selon le procédé de *"détermination du potentiel de défense antiradicalaire",* objet de la demande de brevet français No. 03 12 351 déposée le 22 octobre 2003, en suivant la cinétique de la lyse d'hématies (notamment des hématies de mouton ; il est également possible d'opérer sur sang total ou plasma sanguin) induite par des radicaux libres générés *in situ,* en présence d'un produit selon l'invention à des doses croissantes de 0 mg/L (lot témoin) à 100 mg/L (lots traités), et avec hydrolyse du milieu réactionnel au moyen d'un mélange d'enzymes (β-glucosidase, sulfatase et β- glucuronidase). Selon ce procédé, on mesure en particulier le temps (T½) qui correspond à la lyse de la moitié des cellules concernées, ici les hématies, en fonction de la concentration (en mg/L) du produit de formule I à tester.

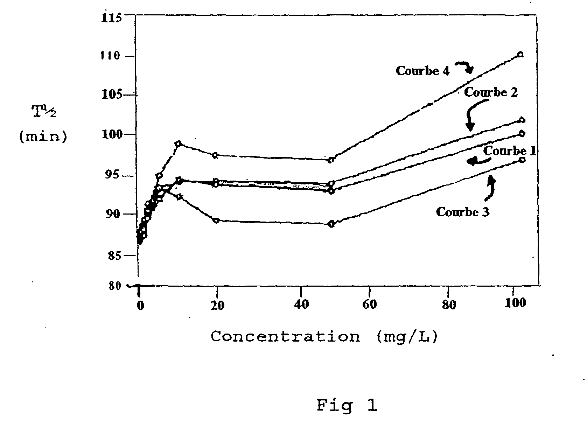

Une partie des résultats obtenus est consignée dans la figure 1 ci-après, dans laquelle la courbe 1 est celle du produit Ex. 1 ; la courbe 2, celle de Ex. 2 ; la courbe 3, celle de Ex. 3 ; et la courbe 4, celle de Ex. 4.

La figure 1 montre que Ex. 4 (i. e. le mélange Ia/IIa 80/20 p/p), qui contient le composé Ia (i. e. Ex. 8) "contaminé" par le composé IIa (i. e. Ex. 2), est plus actif en tant que substance antiradicalaire que Ex. 1, Ex. 2 et Ex. 3.

### Essais H

Des essais complémentaires ont été réalisés avec Ex. 10 et ses constituants (Ex. 5, Ex. 2 et Ex. 3) sur des cellules sanguines humaines [fournies pas EFS (Etablissements Français du Sang)].

Il s'agit de cellules sanguines isolées sur coussin de Ficoll et conservées sous vapeur d'azote liquide. Après décongélation, lesdites cellules sont incubées pendant 24 h à 37 °C avant l'addition des produits de formule 1 à tester. Après nouvelle incubation à 37 °C pendant 24 h ou 48 h, les cellules sont analysées pour apprécier l'éventuelle expression de marqueurs membranaires significatifs, selon le tableau II qui suit.

**Tableau II**

| Analyses du matériel cellulaire | Expression du marqueur membranaire |
|---|---|
| Lymphocytes T | CD3 |
| Lymphocytes T cytotoxiques | CD8 |
| Lymphocytes T "helper" | CD4 |
| Lymphocytes B | CD19 |
| Monocytes/macrophages | CD11c |
| Activations cellulaires | CD69 |
| Surnageants cellulaires | IL-2 |

Comme indiqué dans le tableau II, les surnageants cellulaires ont été analysés pour leur contenu en interleukine 2 (IL-2), produit induisant la prolifération des lymphocytes T, avec ou sans ajout d'un activateur notamment (i) la phyto-hématoglutinine (PHA), qui est un activateur classique, et (ii) un super antigène (SEB), qui induit une interaction entre les molécules de classe II des lymphocytes B avec les récepteurs des lymphocytes T ou TRC mimant de ce fait une présentation d'antigène

On observe deux points majeurs, à savoir :
(1) Ex. 10 et ses constituants, Ex. 5, Ex. 2 et Ex. 3, n'induisent pas la prolifération des cellules sanguines de la réponse immunitaire ; et
(2) Ex. 10, Ex. 5, Ex. 2 et Ex. 3 sont actifs sur ces cellules et vont interférer avec les cascades de signaux entraînant une réponse immunitaire ; l'effet observé semble être immunosuppresseur avec une diminution de production d'anticorps pour les lymphocytes B, une diminution des MHC de classe II pour les cellules dendritiques et une inhibition de la production d'IL-2 (facteur induisant la prolifération lymphocytaire) suite à la stimulation par PHA ou SEB.

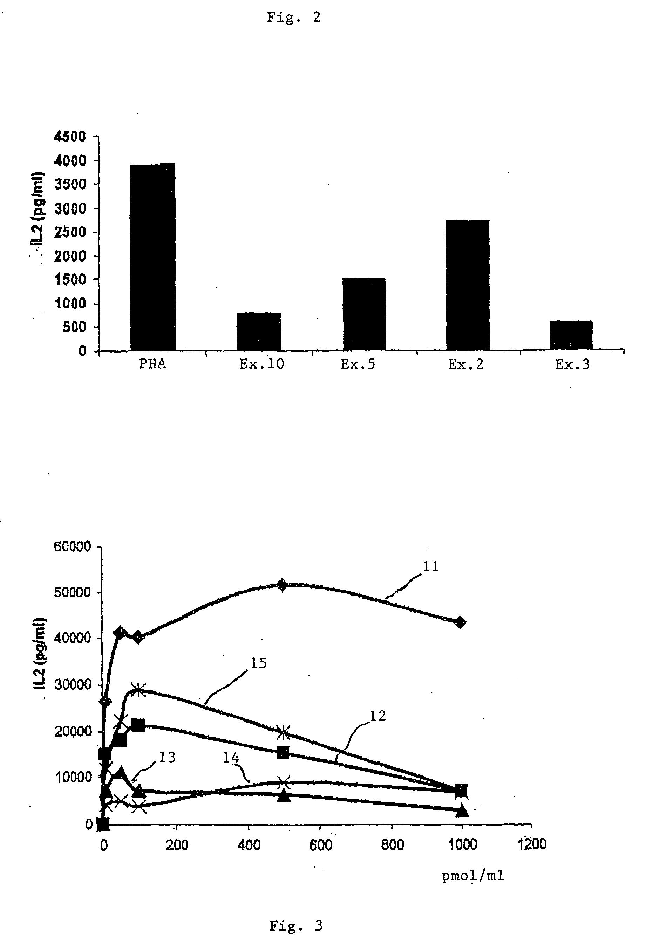

Les figures 2 et 3 montrent l'effet des produits Ex. 10, Ex. 5, Ex. 2 et Ex. 3 sur la sécrétion de IL-2 induite par PHA (figure 2) et, respectivement, par SEB (figure 3). En particulier la figure 3, d'une part, présente la production (exprimée en pg/mL) de IL-2 par rapport à la concentration (exprimée en pmol/mL) en SEB (courbe 11), SEB + Ex. 10 (courbe 12), SEB + Ex. 5 (courbe 13), SEB + Ex. 2 (courbe 14) et SEB + Ex. 3 (courbe 15) et, d'autre part, montre l'effet des produits Ex.10, Ex. 5, Ex. 2 et Ex. 3 sur la stimulation des cellules immunitaires.
En conclusion, les composés de formule IV, et notamment les produits des exemples 1, 4, 8, 9 et 10 sont particulièrement intéressants eu égard à :
- leurs effets immunomodulateurs, notamment vis-à-vis de poussées récentes de sclérose en plaque ;
- leurs effets immunosuppresseurs, notamment illustrés par inhibition de l'activité des stimulants PHA et SEB sur la production de IL-2 ;
- leurs effets anti-blastiques (i.e. par destruction des leucoblastes) et qui sont utiles dans le traitement de la leucémie myéloïde chronique et des leucémies aiguës ;
- leurs effets contre certains cancers; et
- la quasi-absence d'effets secondaires néfastes quand ils sont administrés par voie topique, orale ou injectable.

Chez l'homme adulte, la posologie recommandée pour les produits de formule I, et de préférence les produits de formule IV, est de l'ordre de 50 mg/kg *per os.* Ces produits peuvent également être administrés par voie locale sous forme de gels, pommades; onguents ou lotions ; dans cette éventualité, la forme locale peut contenir de 1 à 5 % en poids de produit de formule I, de formule IV ou d'un de leurs mélanges par rapport au poids de ladite forme locale.

## Revendications

1. Utilisation d'un dérivé de genkwanine ou de sakuranétine, ladite utilisation étant **caractérisée en ce que** l'on fait appel à une substance choisie parmi l'ensemble constitué par
(i) les dérivés osylés de genkwanine ou sakuranétine de formule I : 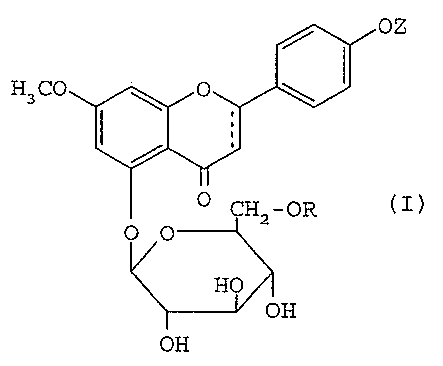 dans laquelle,
le symbole - représente une simple ou une double liaison,
R représente H ou un reste osyle, notamment de structure S¹ ou S² : 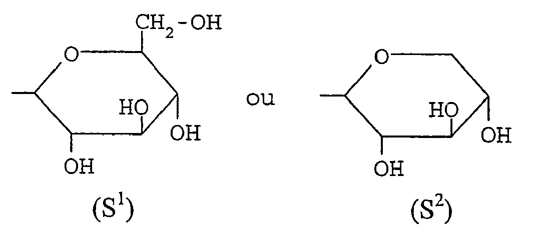
Z représente H, un groupe alkyle en C₁-C₄, acyle en C₁-C₅, ose ou sulfate, et
(ii) leurs mélanges,
en tant qu'ingrédient actif cosmétique ou dermatologique pour l'obtention d'une préparation cosmétique ou dermatologique pour améliorer la texture de la peau.

2. Utilisation d'un dérivé de genkwanine ou de sakuranétine, ladite utilisation étant **caractérisée en ce que** l'on fait appel à une substance choisie parmi l'ensemble constitué par
(i) les dérivés osylés de genkwanine ou sakuranétine de formule I: 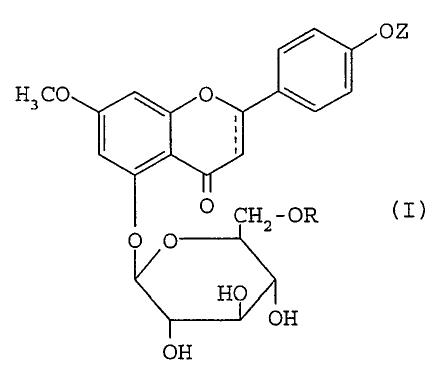 dans laquelle,
le symbole ---- représente une simple ou une double liaison,
R représente H ou un reste osyle, notamment de structure S¹ ou S² : 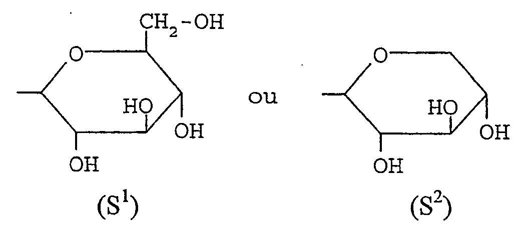
Z représente H, un groupe alkyle en C₁-C₄, acyle en C₁-C₅, ose ou sulfate, et
(ii) leurs mélanges,
en tant qu'ingrédient actif antiradicalaire pour l'obtention d'un médicament destiné à un usage en thérapeutique vis-à-vis des désordres provoqués par les radicaux libres.

3. Utilisation suivant la revendication 1 ou 2, **caractérisée en ce que** ladite substance est choisie parmi l'ensemble constitué par
• les composés de formule IV : 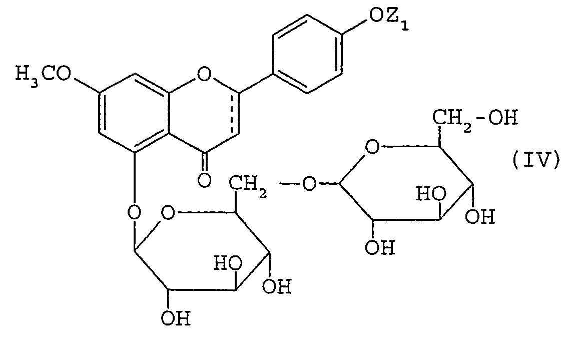 dans laquelle symbole == représente une simple ou une double liaison et Z₁ représente H, un groupe alkyle en C₁-C₄, acyle en C₁-C₅, ose ou sulfate et est avantageusement un groupe alkyle en C₁-C₄ (de préférence le groupe éthyle) ou sulfate (de préférence le groupe SO₃H), et
• leurs mélanges.

4. Utilisation suivant la revendication 1 ou 2, **caractérisée en ce que** ladite substance est choisie parmi l'ensemble constitué par :
• la 5-[O-6-(D-glucopyranosyl)-β-D-glucopyranosyl]oxy-2-(4-éthoxyphényl)-7-méthoxy-4H-1-benzopyran-4-one de formule Ia : 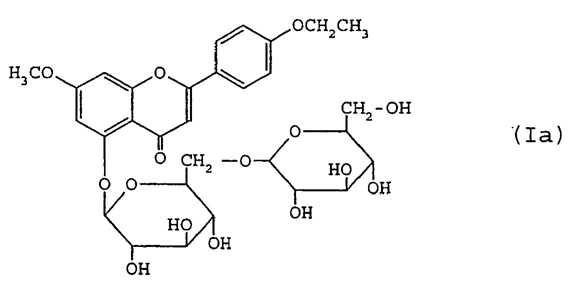
• la 5-O-β-D-primevérosyl-genkwanine de formule IIa : 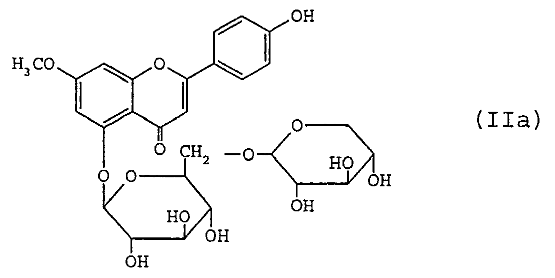
• le pinostrobine-5-glucoside de formule IIIa : 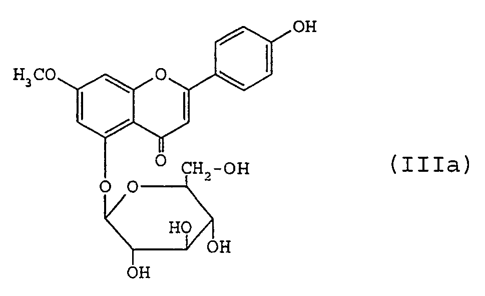
• la 2,3-dihydro-5-[O-6-(D-glucopyranosyl)-β-D-glucopyranosyl]oxy-2-(4-éthoxyphényl)-7-méthoxy-4H-1-benzopyran-4-one de formule Ib : 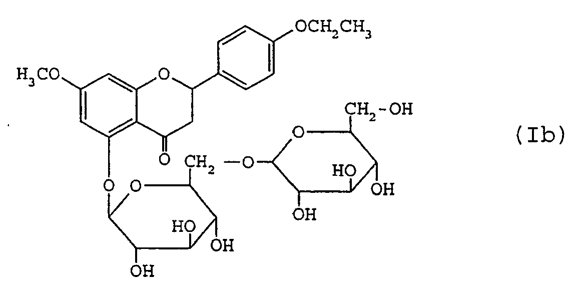
• la 5-O-β-D-primevérosyl-sakuranétine de formule IIb : 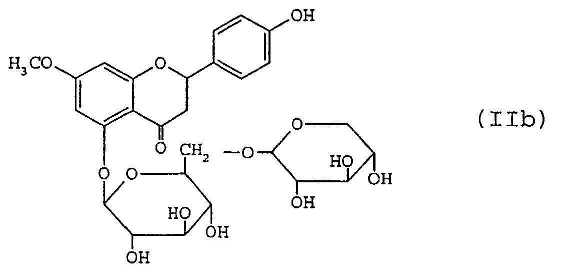
• leurs dérivés où Z est un groupe sulfate (de préférence SO₃H, ou le cas échéant SO₃Na voire SO₃NH₄), et
• leurs mélanges.

5. Dérivé osylé de genkwanine ou de sakuranétine, **caractérisé en ce qu'**il est choisi parmi l'ensemble constitué par
• les composés répondant à la formule générale IV : 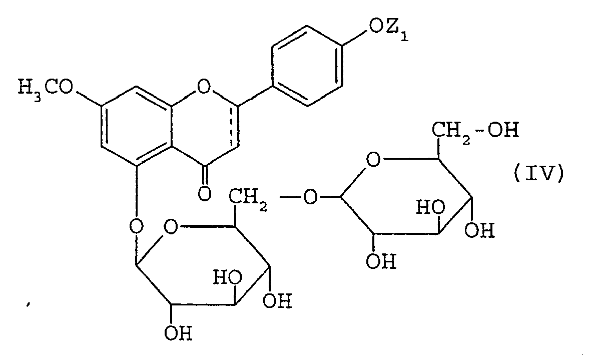 dans laquelle symbole ---- représente une simple ou une double liaison et Z₁ représente H, un groupe alkyle en C₁-C₄, acyle en C₁-C₅, ose ou sulfate et est avantageusement un groupe alkyle en C₁-C₄ (de préférence le groupe éthyle) ou sulfate (de préférence le groupe SO₃H); et,
• leurs mélanges.

6. Dérivé osylé de genkwanine suivant la revendication 5, **caractérisé en ce que** ledit est un composé répondant à la formule Ia :

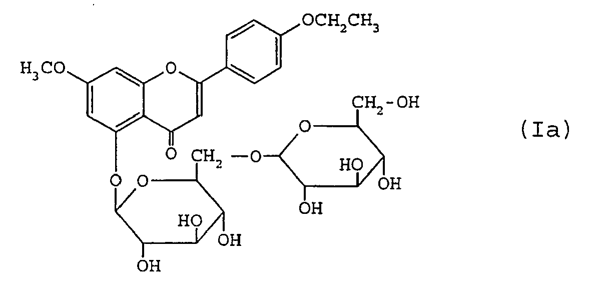

7. Procédé pour la préparation d'un composé de formule I selon la revendication 1 ou 2, ledit procédé étant **caractérisé en ce qu'**il comprend les étapes suivantes :
(i) on extrait la genkwanine, la sakuranétine ou un de leurs osides à partir d'une plante appropriée appartenant à l'ensemble des ***Prunus, Gnidia*** et ***Daphne;***
(ii) on osyle en position 5 l'aglycone avec un ose approprié (si nécessaire après blocage de la fonction OH en position 4' si celle-ci n'est pas protégée) ; et/ou
(iii) on éthérifie (notamment au moyen d'un iodure d'alkyle pour ne pas affecter les groupes OH de la portion sucre), estérifie ou sulfate le groupe 4'-OH de l'oside extrait ou préparé comme indiqué ci-dessus (si nécessaire après déprotection du groupe 4'-OH).

8. Procédé suivant la revendication 7, pour la préparation du composé de formule Ia selon la revendication 4 ou 6, ledit procédé étant **caractérisé en ce qu'**il comprend les étapes consistant à :
(1 °) extraire les racines broyées de ***Daphne gnidium*** avec CH₂Cl₂,
(2°) filtrer pour écarter la solution de chlorure de méthylène, ainsi obtenue, et recueillir le résidu solide que l'on sèche ;
(3°) extraire ledit résidu solide sec, ainsi obtenu, avec CH₃OH ;
(4°) filtrer pour recueillir la solution méthanolique, ainsi obtenue, et écarter le résidu solide résultant ;
(5°) évaporer à sec la solution méthanolique, ainsi recueillie, sous vide, à une température inférieure ou égale à 60 °C, pour obtenir un résidu solide ;
(6°) laver le résidu solide, ainsi obtenu à l'étape (5°), avec de l'eau à une température inférieure ou égale à 60 °C sous agitation, et laisser refroidir ;
(7°) éliminer l'eau de lavage puis reprendre le résidu solide avec CH₃OH ;
(8°) reproduire 3 à 7 fois le cycle des opérations des étapes (5°) à (7°) jusqu'à ce que la dernière eau de lavage soit jaune pâle et limpide ;
(9°) reprendre le résidu sec résultant avec un mélange méthanol-eau 25/2 p/p selon une quantité appropriée pour obtenir un liquide ayant une densité de 0,885 g/mL ;
(10°) laisser reposer ledit liquide à 2-4 °C, de préférence à 3 °C, pendant au moins 2 jours, de préférence pendant 3 jours, et recueillir le précipité formé ;
(11°) laver ledit précipité avec successivement du méthanol puis des mélanges méthanol-éther ayant des teneurs croissantes en éther, jusqu'à ce que le surnageant soit incolore ;
(12°) filtrer le précipité, ainsi obtenu, et le laver plusieurs fois à l'éther, jusqu'à ce que l'éther de lavage soit incolore ;
(13°) filtrer et sécher le produit solide résultant qui est constitué d'un mélange des produits de formules Ia, IIa et IIIa ; et,
(14°) si nécessaire, séparer ledit mélange pour recueillir le produit de formule Ia.

9. Procédé suivant la revendication 8, **caractérisé en ce que** l'extraction de l'étape (1°) est menée à une température de 30-35 °C sous pression atmosphérique ou, le cas échéant, à une température plus élevée sous pression réduite, pendant 3-6 jours, dans un appareil de type Kumagawa ; et **en ce que** l'extraction de l'étape (3°) est effectuée à une température de 45-55 °C, sous pression normale ou, le cas échéant, à une température plus élevée sous pression réduite, dans le même dit appareil, pendant 3-6 jours.

10. Composition cosmétique (a), dermatopharmaceutique (b) ou thérapeutique (c), **caractérisée en ce que** :
(a) la composition cosmétique renferme, en association avec un excipient physiologiquement acceptable par voie topique, au moins un composé de formule I ;
(b) la composition dermatopharmaceutique renferme, en association avec un excipient physiologiquement acceptable notamment par voie topique, au moins un composé de formule 1 ; ou,
(c) la composition thérapeutique renferme, en association avec un excipient physiologiquement acceptable notamment par voie orale ou injectable, au moins un composé de formule IV en tant qu'ingrédient actif immunomodulateur, notamment vis-à-vis de poussées récentes de sclérose en plaque, ou anticancéreux, notamment vis-à-vis de la leucémie myéloïde chronique.

## Claims

1. Use of a genkwanine or sakuranetine derivative, said use being **characterised in that** implementation is made of a substance selected from the group made up of
(i) the osylated derivatives of genkwanine or sakuranetine of formula I: 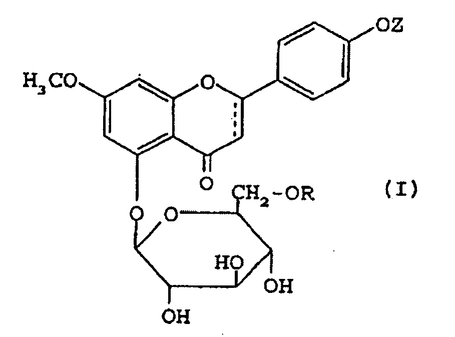 wherein,
the symbol --- represents a single or double bond,
R represents H or an osyl residue, in particular of structure S¹ or S²: 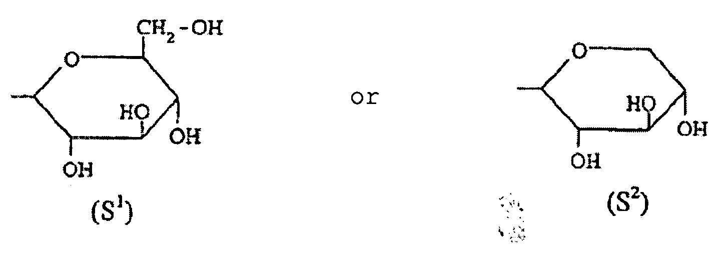
Z represents H, an alkyl group of C₁-C₄, an acyl group of C₁-C₅, an ose group or a sulphate group, and
(ii) their mixtures,
as cosmetic or dermatologic active ingredient for obtaining a cosmetic or dermatologic preparation for improving skin texture.

2. Use of a genkwanine or sakuranetine derivative, said use being **characterised in that** implementation is made of a substance selected from the group made up of
(i) osylated derivatives of genkwanine or sakuranetine of formula I: 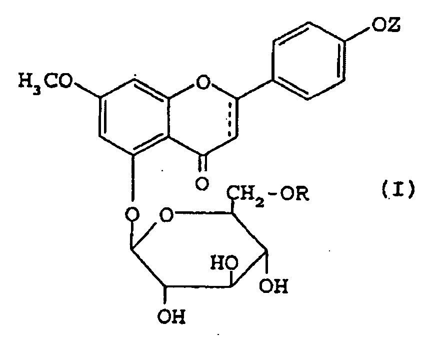 wherein,
the symbol --- represents a single or double bond,
R represents H or an osyl residue, in particular of structure S¹ or S²: 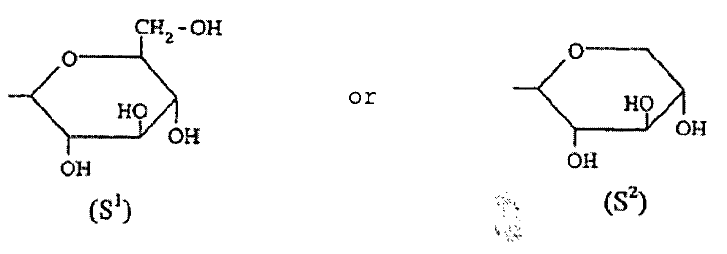
Z represents H, an alkyl group of Cₗ-C₄, an acyl group of C₁-C₅, an ose group or a sulphate group, and
(ii) their mixtures,
as antiradical active ingredient for the obtaining of a medication intended for therapeutic use in disorders caused by free radicals.

3. Use as set forth in claim 1 or 2, **characterised in that** said substance is selected from the group made up of
- the compounds of formula IV: 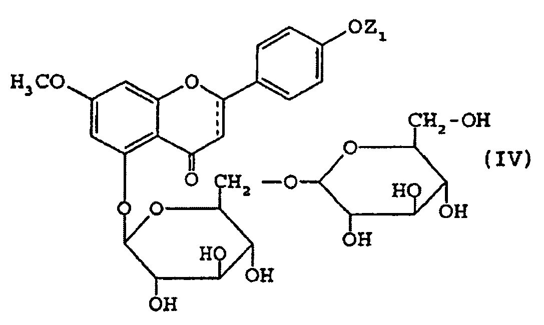 wherein the symbol --- represents a single or double bond and Z₁ represents H, an alkyl group of C₁-C₄, an acyl group of C₁-C₅, an ose group or a sulphate group and is advantageously an alkyl group of C₁-C₄ (more preferably the ethyl group) or a sulphate group (more preferably the SO₃H group), and
- their mixtures.

4. Use as set forth in claim 1 or 2, **characterised in that** said substance is selected from the group made up of:
- 5-[O-6-(D-glucopyranosyl)-β-D-glucopyranosyl]oxy-2-(4-ethoxyphenyl)-7-methoxy-4H-1-benzopyran-4-one of formula Ia: 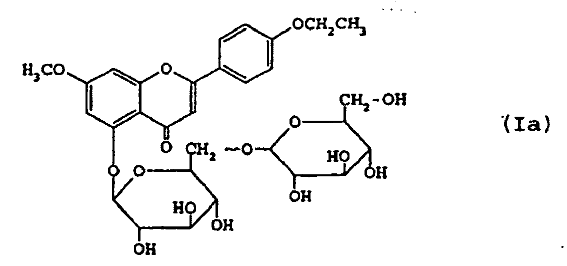
- 5-0-β-D-primeverosyl-genkwanine of formula IIa: 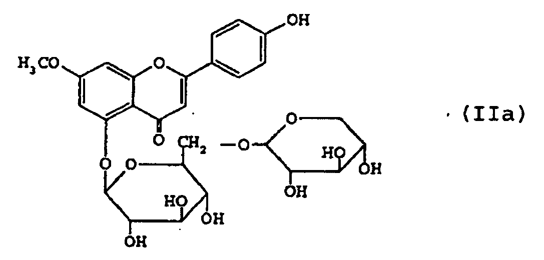
- pinostrobine-5-glucoside of formula IIIa: 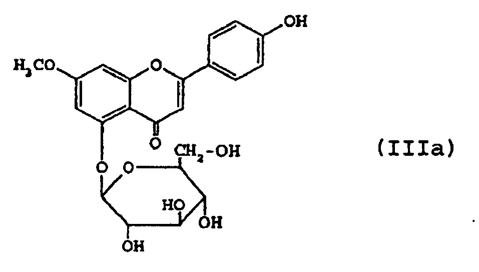
- 2,3-dihydro-5-[O-6-(D-glucopyranosyl)-β-D-glucopyranosyl]oxy-2-(4-ethoxyphenyl)-7-methoxy-4H-1-benzopyran-4-one of formula Ib: 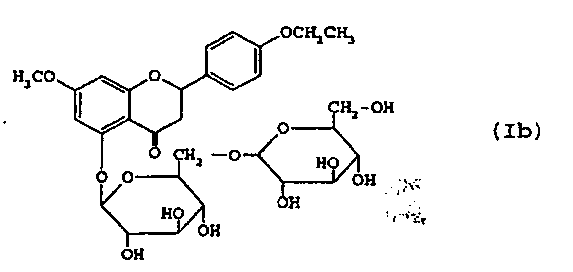
- 5-O-β-D-primeverosyl-sakuranetine of formula IIb: 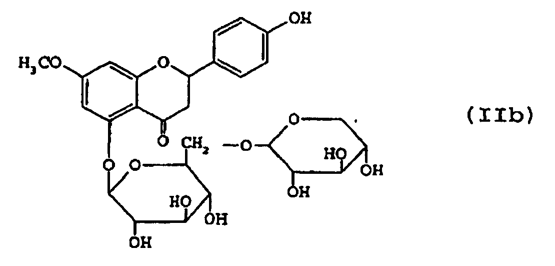
- their derivatives in which Z is a sulphate group (more preferably SO₃H, or if need be SO₃Na or even SO₃NH₄), and
- their mixtures.

5. Osylated derivative of genkwanine or sakuranetine, **characterised in that** it is selected from the group made up of
- compounds conforming to general formula IV: 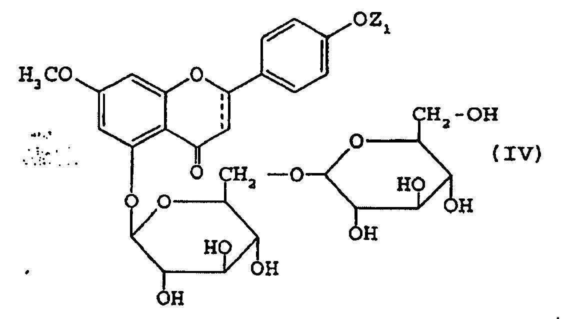 wherein the symbol --- represents a single or double bond and Z₁ represents H, an alkyl group of C₁-C₄, an acyl group of C₁-C₅, an ose group or a sulphate group and is advantageously an alkyl group of C₁-C₄ (more preferably the ethyl group) or a sulphate group (more preferably the SO₃H group); and,
- their mixtures.

6. Osylated derivative of genkwanine as set forth in claim 5, **characterised in that** said derivative is a compound conforming to formula Ia:

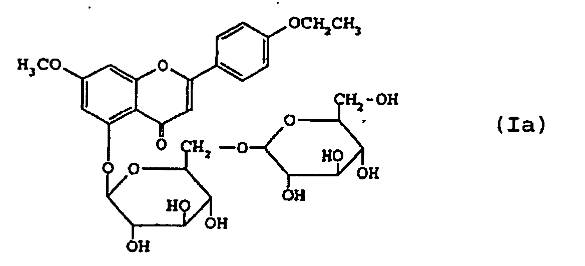

7. Method for the preparation of a compound of formula I as set forth in claim 1 or 2, said method being **characterised in that** it comprises the following steps:
(i) genkwanine, sakuranetine or one of their osides is extracted from an appropriate plant belonging to the ***Prunus, Gnidia* and *Daphne*** group;
(ii) the aglycone is osylated in position 5 with an appropriate ose (as required after blockage of the OH function in position 4' if this is not protected); and/or
(iii) the 4'-OH group of the oside extracted or prepared as indicated above is etherified (in particular by means of an alkyl iodide in order to not affect the OH groups of the sugar portion), esterified or sulphated (as required after deprotection of the 4'-OH group).

8. Method as set forth in claim 7, for the preparation of the compound of formula Ia as set forth in claim 4 or 6, said method being **characterised in that** it comprises the steps consisting in:
(1^{st}) extract the ground roots of ***Daphne gnidium*** with CH₂Cl₂,
(2^{nd}) filter to separate the methylene chloride solution, thus obtained, and collect the solid residue which is dried;
(3^{rd}) extract said dry solid residue, thus obtained, with CH₃OH;
(4^{th}) filter to collect the methanolic solution, thus obtained, and separate the resultant solid residue;
(5^{th}) evaporate to dryness the methanolic solution, thus collected, under vacuum, at a temperature less than or equal to 60°C, to obtain a solid residue;
(6^{th}) wash the solid residue, thus obtained in step (5^{th}), with water at a temperature less than or equal to 60°C under agitation, and allow it to cool;
(7^{th}) discard the wash water and then take up the solid residue again with CH₃OH;
(8^{th}) repeat the cycle of operations of steps (5^{th}) to (7^{th}) 3 to 7 times until the last wash water is pale yellow and clear;
(9^{th}) take up the resultant dry residue again with a methanol-water 25/2 p/p mixture in an appropriate quantity to obtain a liquid with a density of 0.885 g/mL;
(10^{th}) allow said liquid to sit at 2-4 °C, more preferably at 3 °C, for at least 2 days, more preferably for 3 days, and collect the precipitate formed;
(11^{th}) wash said precipitate successively with methanol and then methanol-ether mixtures with increasing ether contents, until the supernatant is colourless;
(12^{th}) filter the precipitate thus obtained, and wash it several times with ether, until the ether wash is colourless;
(13^{th}) filter and dry the resultant solid product which is made up of a mixture of the products of formulas Ia, IIa and IIIa; and,
(14^{th}) as required, separate said mixture in order to collect the product of formula Ia.

9. Method as set forth in claim 8, **characterised in that** the extraction of step (1^{st}) is conducted at a temperature of 30-35 °C under atmospheric pressure or, if need be, at a higher temperature under reduced pressure, for 3-6 days, in a Kumagawa apparatus; and **in that** the extraction of step (3^{rd}) is carried out at a temperature of 45-55 °C, under normal pressure or, if need be, at a higher temperature under reduced pressure, in the same said apparatus, for 3-6 days.

10. Cosmetic (a), dermatopharmaceutic (b) or therapeutic (c) composition, **characterised in that**:
(a) the cosmetic composition contains, in association with an excipient that is physiologically acceptable by the topical route, at least one compound of formula I;
(b) the dermatopharmaceutic composition contains, in association with an excipient that is physiologically acceptable in particular by the topical route, at least one compound of formula I; or,
(c) the therapeutic composition contains, in association with an excipient that is physiologically acceptable in particular by the oral or injection route, at least one compound of formula IV as immunomodulating active ingredient, in particular in recent episodes of multiple sclerosis, or as anticancer active ingredient, in particular in chronic myeloid leukaemia.

## Patentansprüche

1. Verwendung eines Genkwanin- oder Sakuranetinderivats, wobei die besagte Verwendung **dadurch gekennzeichnet ist, dass** auf eine Substanz zurückgegriffen wird, die ausgewählt ist aus der Gruppe, gebildet von
(i) den glycosylierten Genkwanin oder Sakuranetinderivaten von Formel I: 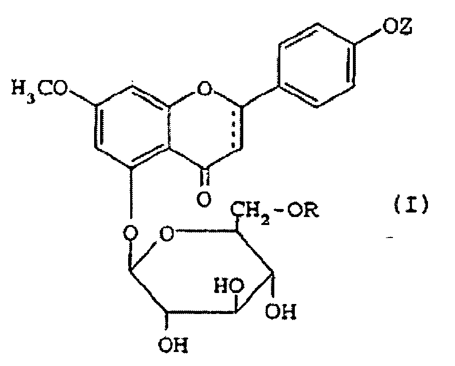 wobei
das Symbol ----- eine Einfach- oder Doppelbindung darstellt,
R H oder einen Glycosylrest darstellt, vor allem der Struktur S¹ oder S²: 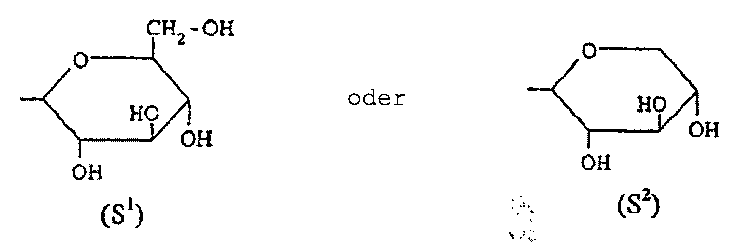
Z H, eine Alkyl- bei C₁-C₄, Acyl- bei C₁-C₅, Glycose- oder Sulfatgruppe darstellt, und
(ii) ihren Gemischen
als kosmetisch oder dermatologisch aktiver Inhaltsstoff zur Herstellung einer kosmetischen oder dermatologischen Zubereitung zur Verbesserung der Textur der Haut.

2. Verwendung eines Genkwanin- oder Sakuranetinderivats, wobei die besagte Verwendung **dadurch gekennzeichnet ist, dass** auf eine Substanz zurückgegriffen wird, die ausgewählt ist aus der Gruppe, gebildet von
(i) den glycosylierten Genkwanin- oder Sakuranetinderivaten von Formel I: 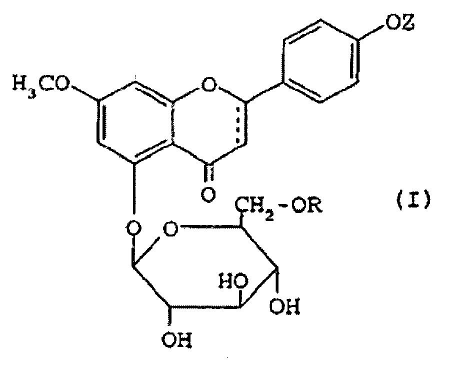 wobei
das Symbol ----- eine Einfach- oder Doppelbindung darstellt,
R H oder einen Glycosylrest darstellt, vor allem der Struktur S¹ oder S²: 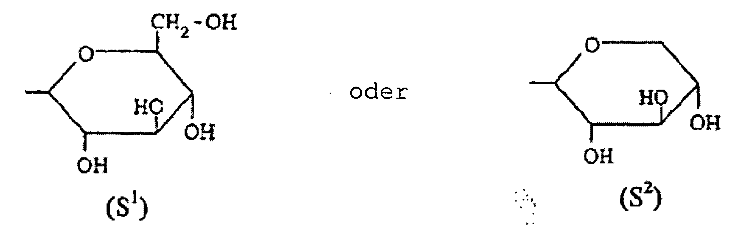
Z H, eine Alkyl- bei C₁-C₄, Acyl- bei C₁-C₅, Glucose- oder Sulfatgruppe darstellt, und
(ii) ihren Gemischen
als gegen Radikale aktiver Inhaltsstoff zur Herstellung eines Medikaments, das für eine therapeutische Anwendung gegenüber von freien Radikalen verursachten Unregelmäßigkeiten bestimmt ist.

3. Verwendung nach Anspruch 1 oder 2, **dadurch gekennzeichnet, dass** die besagte Substanz ausgewählt ist aus der Gruppe, gebildet von
• den Verbindungen von Formel IV: 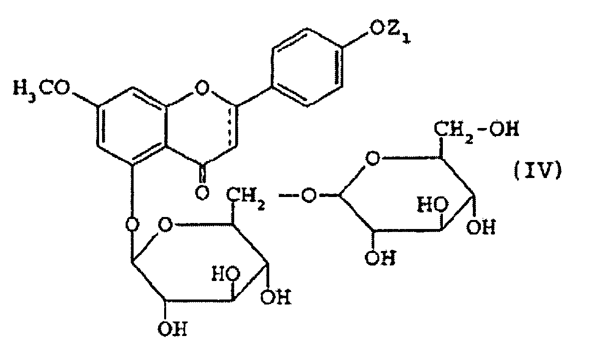 wobei das Symbol ----- eine Einfach- oder Doppelbindung darstellt und Z₁ H, eine Alkyl-bei C₁-C₄, Acyl- bei C₁-C₅, Glycose- oder Sulfatgruppe darstellt und in vorteilhafter Weise eine Alkylgruppe bei C₁-C₄ (vorzugsweise die Ethylgruppe) oder Sulfatgruppe (vorzugsweise die Gruppe SO₃H) ist, und
• ihren Gemischen.

4. Verwendung nach Anspruch 1 oder 2, **dadurch gekennzeichnet, dass** die besagte Substanz ausgewählt ist aus der Gruppe, gebildet von:
• dem 5-[0-6-(D-Glucopyranosyl)-β-D-Glucopyranosyl]oxy-2-(4-Ethoxyphenyl)-7-Methoxy-4H-1-Benzopyran-4-on von Formel Ia: 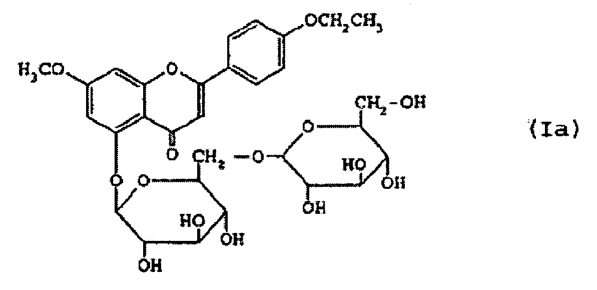
• dem 5-O-β-D-Primeverosyl-Genkwanin von Formel IIa: 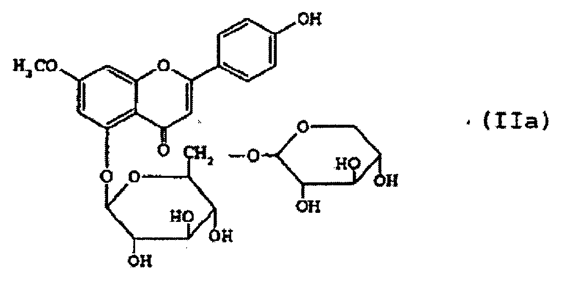
• dem Pinostrobin-5-Glucosid von Formel IIIa: 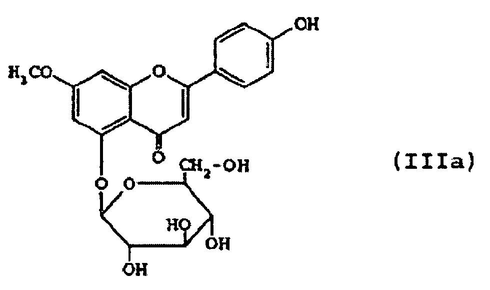
• dem 2,3-Dihydro-5-[O-6-(D-Glucopyranosyl)-β-D-Glucopyranosyl]oxy-2-(4-Ethoxyphenyl)-7-Methoxy-4H-1-Benzopyran-4-on von Formel Ib: 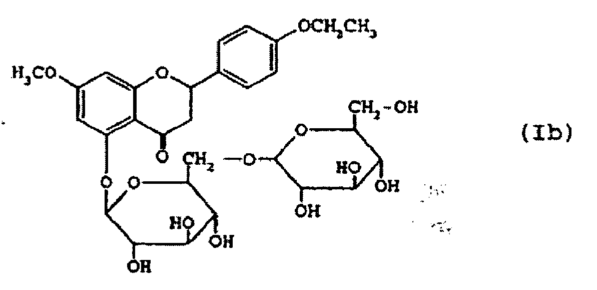
• dem 5-0-β-D-Primeverosyl-Sakuranetin von Formel IIb: 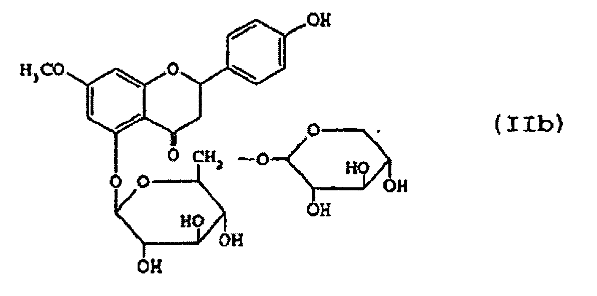
• ihren Derivaten, wobei Z eine Sulfatgruppe (vorzugsweise SO₃H oder gegebenenfalls SO₃Na, ja sogar SO₃NH₄) ist, und
• ihren Gemischen.

5. Glycosyliertes Genkwanin- oder Sakuranetinderivat, **dadurch gekennzeichnet, dass** es ausgewählt ist aus der Gruppe, gebildet von:
• den Verbindungen, die der allgemeinen Formel IV entsprechen 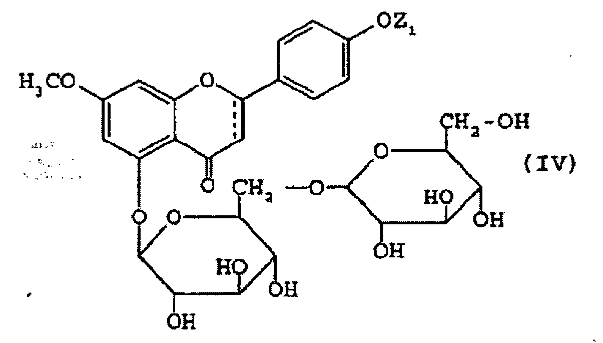 wobei das Symbol ----- eine Einfach- oder Doppelbindung darstellt und Z₁ H, eine Alkyl- bei C₁-C₄, Acyl- bei C₁-C₅, Glycose- oder Sulfatgruppe darstellt und in vorteilhafter Weise eine Alkylgruppe bei C₁-C₄ (vorzugsweise die Ethylgruppe) oder Sulfatgruppe (vorzugsweise die Gruppe SO₃H) ist, und
• ihren Gemischen.

6. Glycosyliertes Genkwaninderivat nach Anspruch 5, **dadurch gekennzeichnet, dass** das Besagte eine Verbindung ist, die der Formel Ia entspricht:

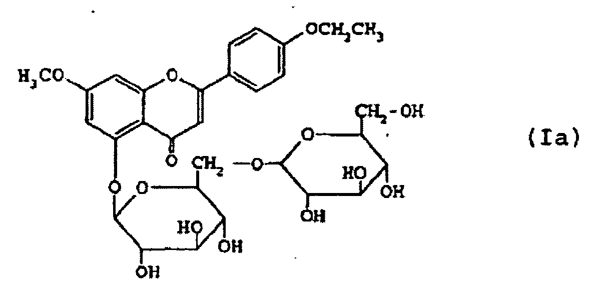

7. Verfahren zur Zubereitung einer Verbindung von Formel I nach Anspruch 1 oder 2, wobei das besagte Verfahren **dadurch gekennzeichnet ist, dass** es die folgenden Schritte umfasst:
(i) Extraktion des Genkwanins, des Sakuranetins oder eines ihrer Glycoside aus einer geeigneten Pflanze, die zur Gruppe der Prunus, Gnidia und Daphne gehört,
(ii) Glycosylierung von Aglycon bei Position 5 mit einer geeigneten Glycose (wenn notwendig, nach Blockierung der Funktion OH bei Position 4', wenn diese nicht geschützt ist); und/oder
(iii) Veretherung (vor allem mit einem Alkyliodid, um die OH-Gruppen des Zuckerabschnitts nicht zu beeinträchtigen), Veresterung oder Sulfatierung der 4'-OH-Gruppe des extrahierten oder zubereiteten Glycosids, wie oben angegeben (wenn notwendig, nach Deprotektion der 4'-OH-Gruppe).

8. Verfahren nach Anspruch 7 für die Zubereitung der Verbindung von Formel Ia nach Anspruch 4 oder 6, wobei das besagte Verfahren **dadurch gekennzeichnet ist, dass** es die Schritte umfasst, die bestehen aus:
(1.) Extraktion der zerkleinerten Wurzeln von Daphne gnidium mit CH₂Cl₂,
(2.) Filtration, um die dabei hergestellte Chlormethylenlösung abzuscheiden, und Auffangen des festen Rückstands, der getrocknet wird;
(3.) Extraktion des besagten, derart hergestellten trockenen festen Rückstands mit CH₃0H;
(4.) Filtration, um die dabei hergestellte Methanollösung aufzufangen, und Abscheiden des resultierenden festen Rückstands;
(5.) Trockenverdampfung der derart aufgefangenen Methanollösung im Vakuum bei einer Temperatur unter oder gleich 60 °C, um einen festen Rückstand herzustellen;
(6.) Waschen des in Schritt (5.) hergestellten festen Rückstands mit Wasser bei einer Temperatur unter oder gleich 60 °C unter Rühren, und abkühlen lassen;
(7.) Entfernung des Waschwassers, danach Aufnahme des festen Rückstands mit CH₃OH;
(8.) 3- bis 7-malige Wiederholung des Zyklus der Arbeitsgänge der Schritte (5.) bis (7.), bis das letzte Waschwasser blassgelb und klar ist;
(9.) Aufnahme des resultierenden trockenen Rückstands mit einem Methanol-Wasser-Gemisch 25/2 p/p in einer geeigneten Menge, um eine Flüssigkeit mit einer Dichte von 0,885 g/mL herzustellen;
(10.) die besagte Flüssigkeit bei 2-4 °C, vorzugsweise bei 3 °C, mindestens 2 Tage lang, vorzugsweise 3 Tage lang, ruhen lassen, und Aufnahme des gebildeten Präzipitats;
(11.) Waschen des besagten Präzipitats mit zunächst Methanol und danach mit Methanol-Ether-Gemischen mit ansteigendem Ethergehalt, bis der Überstand farblos ist;
(12.) Filtration des derart hergestellten Präzipitats und mehrmaliges Waschen desselben in Ether, bis der Waschether farblos ist;
(13.) Filtration und Trocknung des resultierenden Feststoffs, der von einem Gemisch der Produkte der Formeln Ia, IIa und IIIa gebildet wird; und
(14.) sofern notwendig, Separierung des besagten Gemischs, um das Produkt von Formel Ia aufzufangen.

9. Verfahren nach Anspruch 8, **dadurch gekennzeichnet, dass** die Extraktion von Schritt (1.) bei einer Temperatur von 30-25 °C unter atmosphärischem Druck durchgeführt wird oder gegebenenfalls bei einer höheren Temperatur unter reduziertem Druck 3-6 Tage lang in einem Apparat vom Typ Kumagawa; und **dadurch**, dass die Extraktion von Schritt (3.) bei einer Temperatur von 45-55 °C unter normalem Druck durchgeführt wird oder gegebenenfalls bei einer höheren Temperatur unter reduziertem Druck in demselben besagten Apparat 3-6 Tage lang.

10. Kosmetische (a), dermatopharmazeutische (b) oder therapeutische (c) Zusammensetzung, **dadurch gekennzeichnet, dass**:
(a) die kosmetische Zusammensetzung in Kombination mit einem auf topischem Wege physiologisch akzeptablen Trägermittel mindestens eine Verbindung von Formal I einschließt;
(b) die dermatopharmazeutische Zusammensetzung in Kombination mit einem vor allem auf topischem Wege physiologisch akzeptablen Trägermittel mindestens eine Verbindung von Formal I einschließt; oder
(c) die therapeutische Zusammensetzung in Kombination mit einem vor allem auf oralem oder injizierbarem Wege physiologisch akzeptablen Trägermittel mindestens eine Verbindung von Formel IV als Inhaltsstoff einschließt, der immunmodulierend aktiv ist, vor allem gegenüber neuen Schüben von Multipler Sklerose, oder antikarzinogen, vor allem gegenüber der chronisch-myeloischen Leukämie.
